# EUROPEAN PATENT APPLICATION

(11) **EP 1 044 663 A2**
(43) Date of publication of application: **18.10.2000**
(21) Application number: 00830278.8
(22) Date of filing: 12.04.2000
(51) Int. Cl.: A61F 2/04

(54) **Intrahepatic endoprosthesis**

(30) Priority: 14.04.1999 IT MI990771
(71) Applicant: Pasquinucci, Marco, 50029 Impruneta FI (IT); Quaretti, Pietro, 43025 Palanzano (PR) (IT)
(72) Inventor: Pasquinucci, Marco, 50029 Impruneta FI (IT); Quaretti, Pietro, 43025 Palanzano (PR) (IT)
(74) Representative: Pizzoli, Antonio

(57) **Abstract**

Intrahepatic endoprosthesis comprising at least one tubular net (2), wherein the end portions (3, 4) of said tubular net (2) are outwardly flared, at least one end portion (3) and a portion of said tubular net (2) included between said end portions (3, 4) are joined to a tube (1; 1a, 1b) of biocompatible material having substantially the same diameter of said tubular net (2). The present invention also relates to a cross-section reducer which can be used in combination with said endoprosthesis.

## Description

The present invention relates to an intrahepatic endoprosthesis, and particularly to an endoprosthesis commonly known with the English name *stent,* which can be used for a type of surgical radiology procedure known with the English name *transjugular intrahepatic portosystemic shunt,* from which the acronym TIPS (or TIPSS) originates. The present invention also relates to a cross-section reducer which can be used in combination with such an endoprosthesis.

The TIPS surgery, introduced in clinical use at the end of the 80's, is known to consist in the insertion at the intrahepatic level, particularly by percutaneous right internal jugular route, one or several endoprostheses formed of tubular metal nets having a diameter generally included between 8 mm and 12 mm, which are arranged between a suprahepatic branch and a portal branch in order to derive the portal blood flow and thus to annihilate the resistance caused by the liver parenchyma. Therefore, this operation enables pressure to be reduced by accelerating the blood flow in the case of portal hypertension, which can be complicated for example by hemorrages of esophageal or gastric varices, refractory ascites, relapsing hydrothorax or hepatorenal syndromes. The TIPS surgery also provides for a low incidence of infra-procedural complications and a higher effectiveness with respect to similar surgical operations, generally based on splenorenal or portocaval derivation.

The analysis of the course of several patients subjected to the TIPS surgery proved the effectiveness of the treatment, however it also pointed out an incidence, which many consider to be high, of stenosis and/or occlusion of the endoprosthesis, which, although can be corrected per cutem through angioplasty or insertion of a new endoprosthesis, involve the need of a severe patient control in order to individuate in useful time possible clues of an incorrect operation of the endoprosthesis itself. Clinical and anatopatological studies proved that the stenosis seats which impede a correct operation of the endoprosthesis are mainly localized at the endoprosthesis center or at the end thereof which is turned towards the suprahepatic vein. Further, it was ascertained that the stenosis at the endoprosthesis center is caused by bile passing among bile channels damaged during the endoprosthesis insertion, thus causing a so-called bile-venous fistula. On the contrary, the stenosis at a suprahepatic branch is due to excessive hyperplasia caused by the reaction of the vascular endothelium to the turbulence of the blood flow which is produced by the union of the high velocity flow of portal origin with the sensibly slower suprahepatic vein flow.

Another drawback of this type of known endoprosthesis consists in the possible operation excess, due for example to a wrong estimation of the diameter of the endoprosthesis with respect to the hepatic functionality, so as to determine in some cases a encephalopathy of disabling grade or a worsening of the hepatic insufficiency, even incompatible with the patient's survival. In these cases, it is necessary to occlude the endoprosthesis by means of embolus-forming presidium, with resulting risk of a sudden recidivation of the portal hypertension.

Therefore, object of the invention is providing an endoprosthesis suitable to eliminate the above mentioned drawbacks. Said object is achieved by means of an endoprosthesis whose main features are specified in the first claim and other features are specified in the following claims.

Thanks to the biocompatible tissue which is provided with, the endoprosthesis according to the present invention prevents the formation of bile-venous or arterio-venous fistulae, so that the blood flow in the endoprosthesis of the adjacent intraparenchymal anatomic structures is isolated. Besides, the flared end portion of the endoprosthesis, intended to be arranged at a suprahepatic branch, allows the velocity and therefore the turbulence in the output flow to be advantageously reduced. In fact, it has been verified that a flow reduction between 30% and 50% is obtained by passing from a cross-section of 9-10 mm of diameter to a section of 12 mm of diameter. The flared end portion of the endoprosthesis intended to be arranged at a portal branch allows to obtain a better stability and to avoid the risk of dangerous endoprosthesis migrations.

Particularly, the flared end portion of the endoprosthesis intended to be arranged at a portal branch is preferably uncoated, that is, free from the coating formed of the biocompatible tissue tube. By this measure, the epathopetal portal blood is advantageously allowed to flow not only towards the endoprosthesis, but also towards the contiguous portal branches, resulting in an improvement of the liver functions.

According to a particular aspect of the invention, the weft of the meshes in the end portions of the tubular net is wider with respect to the weft of the meshes comprised between the portions themselves. By this measure, the risk of haemolysis because of mechanical breakage due to the impact of the red corpuscles against the tubular net meshes is reduced, in particular in the flared and uncoated end portion intended to be arranged at a portal branch.

According to another particular aspect of the invention, the tubular net can be arranged inside or outside the biocompatible tissue net, as a function of the features of adhesion to the hepatic parenchyma required by the patient's pathology.

A preferred embodiment of the endoprosthesis according to the present invention is advantageously formed of two members that can be connected telescopically, so as to compose a length-extensible endoprosthesis. By this measure it is possible to precisely adapt the length of the endoprosthesis to the intraparenchymal hepatic means without resorting to complicated designing studies before the surgery.

Another advantage of the endoprosthesis according to the present invention consist in that, by virtue of the structure and the physical features of the material which the tubular net is made with, the shape and disposition of the endoprosthesis are perfectly correct even after the compression required for the positioning thereof, so avoiding risks of kink.

A further advantage of the endoprosthesis according to the present invention consist in that the minimum diameter thereof can be easily reduced by the insertion of particular reducers of the cross-section which have features similar to those of the endoprosthesis itself and can be used also in combination with endoprosthesis of a known type, once the endoprosthesis has been inserted into the patient's liver, so as to reduce the intrahepatic flow without the risk of recidivation of the portal hypertension.

Finally, the tubular net of the endoprosthesis is preferably and advantageously made in a nickel - titanium alloy known with the name nitinol which, beside being biocompatible, is provided with both high features of elasticity and a so-called "shape-memory" which allows the structure made with this alloy to return perfectly to a predetermined shape, as a function of the temperature whereto said structure was made, for instance to 37°C, that is the human body temperature.

Further advantages and features of the endoprosthesis according to the present invention will appear from the following detailed description of some embodiments thereof with reference to the accompanying drawings wherein:
- figure 1 shows a side view of a first embodiment of the endoprosthesis according to the present invention;
- figure 2 shows a side view in longitudinal section of the endoprosthesis of figure 1;
- figure 3 shows a side view of a second embodiment of the endoprosthesis according to the present invention;
- figure 4 shows a side view in longitudinal section of the endoprosthesis of figure 3;
- figure 5 shows a side view of a third embodiment of the endoprosthesis according to the present invention;
- figure 6 shows a side view in longitudinal section of the endoprosthesis of figure 5;
- figure 7 shows a side view of a first member of a fourth and preferred embodiment of the endoprosthesis according to the present invention;
- figure 8 shows a side view of a second member which can be coupled to the member of figure 7;
- figure 9 shows a side view of the members of figure 7 and 8 coupled so as to form the fourth embodiment of the endoprosthesis according to the present invention;
- figure 10 shows a side view of a first embodiment of a cross-section reducer which can be used with an endoprosthesis according to the present invention;
- figure 11 shows a side view in longitudinal section of the cross-section reducer of figure 10;
- figure 12 shows a side view of a second embodiment of a cross-section reducer which can be used with an endoprosthesis according to the present invention;
- figure 13 shows a side view in longitudinal section of the cross-section reducer of figure 12;
- figure 14 shows a partial side view of a metal wire used for the manufacture of the endoprosthesis of figure 1;
- figure 15 shows a partial side view of a metal wire used for the manufacture of the endoprosthesis of figure 3;
- figure 16 shows a partial side view of a metal wire used for the manufacture of the endoprosthesis of figure 5;
- figure 17 shows a schematic side view of the member of figure 7 inserted into a patient's liver; and
- figure 18 shows a schematic side view of the endoprosthesis of figure 9 inserted into a patient's liver.

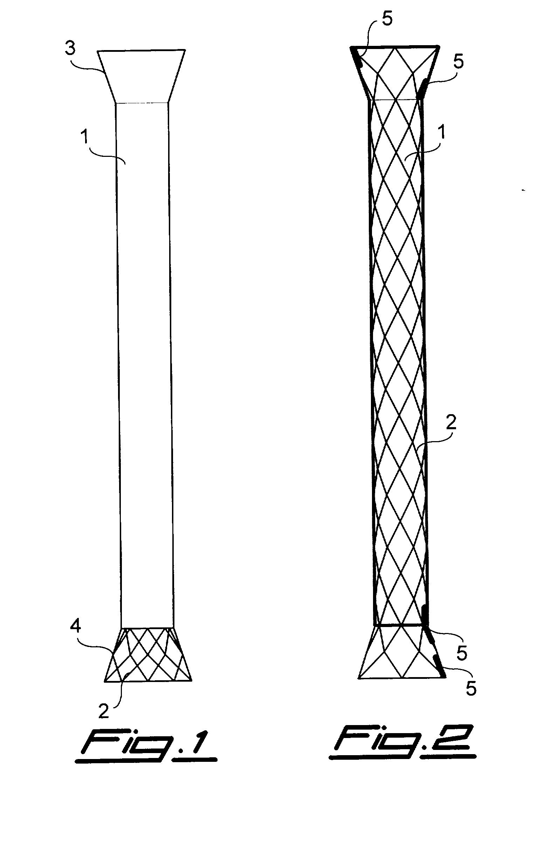

With reference to figures 1 and 2, the first embodiment of the endoprosthesis according to the present invention is shown to comprise a tube 1 of biocompatible tissue, particularly a low porosity tissue in synthetic material such as PTFE or polyester. On the internal surface of tube 1 is fastened, for example by means of microscopic sutures, a semirigid tubular net 2 provided with rhomboid-shaped meshes made with stainless steel 316L or preferably with an alloy with "shape memory" properties, such as the nickel-titanium alloy known with the name nitinol. The tubular net preferably has, in the expanded condition, a diameter included between 8 and 12 mm and a length included between 10 and 12 cm.

The end portions 3, 4 of the tubular net 2, preferably 2 to 3 cm long, are suitably outwards flared so that said portions have a maximum diameter included between 12 and 13 mm. Resultingly, the mesh weft at end portions 3, 4 of tubular net 2 is wider than the weft of the meshes included between the portions themselves. Further, while the end portion 3 which is intended to be arranged at a suprahepatic branch is coated by the tube 1 of biocompatible tissue, end portion 4 which is intended to be arranged at a portal branch is not coated by tube 1 of biocompatible tissue, so that tubular net 2 remains uncoated in the latter end portion. A multiplicity of signaling members 5 which, being opaque to X-rays, are useful for localizing the endoprosthesis during its positioning, are arranged at the ends and at the borders between end portions 3, 4 and the central portion of tubular net 2.

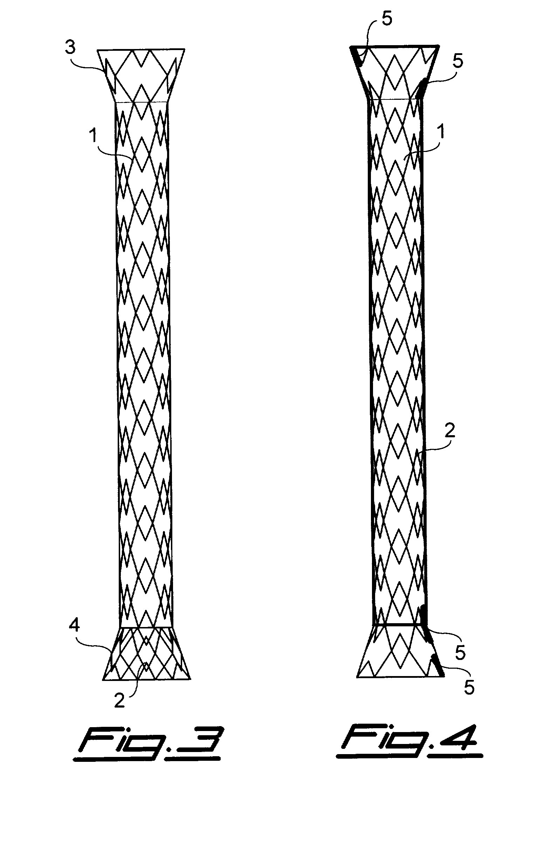

Now, with reference to figures 3 and 4, the second embodiment of the endoprosthesis according to the present invention is also shown to comprise a tube 1 of biocompatible tissue joined to a metal tubular net 2. However, in this embodiment tubular net 2 is arranged externally of tube 1 and the meshes of net 2 are not rhomboidal-shaped, but form a more complex pattern suitable for providing a better support for tissue tube 1, elasticity and flexibility of the endoprosthesis being equal. In facts, in both the above mentioned embodiments tubular net 2 is provided with high features of elasticity and flexibility, so as to prevent risks of endoprosthesis kinks at the bends, as well as the unintentional shortening during the transition from the compressed to the expanded condition.

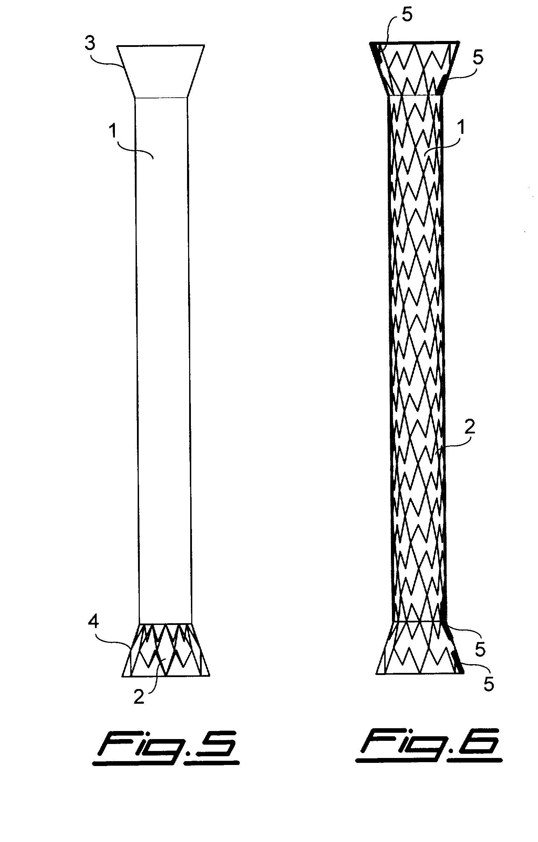

Figures 5 and 6 show the third embodiment of the endoprosthesis according to the present invention, wherein tubular net 2 is arranged inside tube 1 and the meshes of net 2 are provided with a further complex pattern suitable for obtaining a support of tissue tube 1 of intermediate type with respect to the first and second embodiment.

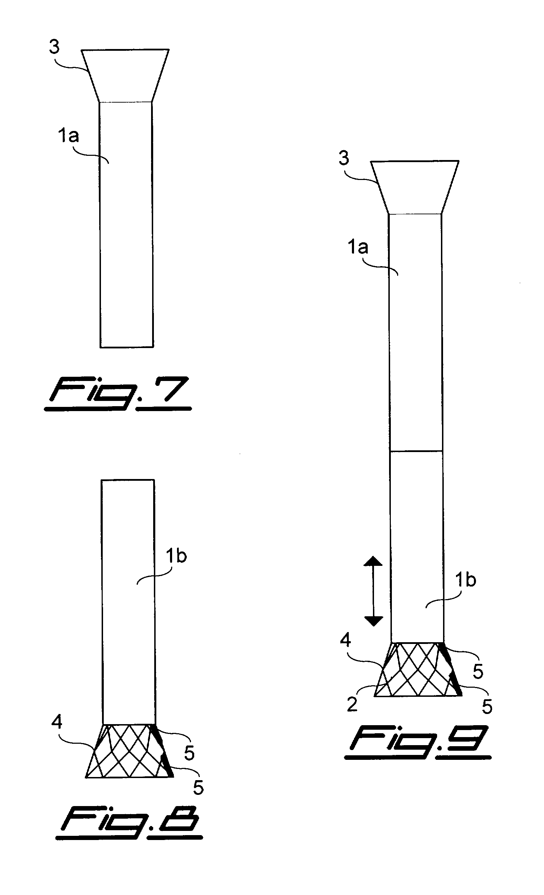

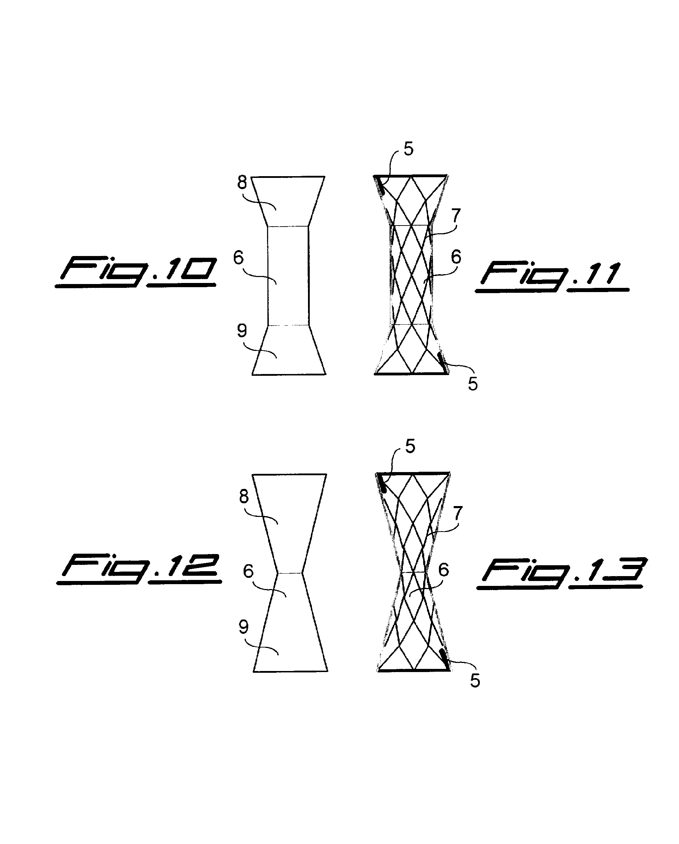

With reference to figures 7 to 9, there is shown that the fourth and preferred embodiment of the endoprosthesis according to the present invention is formed of two members which can be coupled together telescopically as in figure 9, each of which comprises a tube la, 1b of biocompatible tissue, which is joined to a tubular net with rhomboidal meshes and is provided with a single flared end portion 3, 4 so that by coupling these members an endoprosthesis similar to that of the first embodiment, but being also extensible in length, can be obtained. For such a purpose, the diameter of the member of figure 7 is slightly larger than that of the member of figure 8, but obviously in other embodiments of the present invention the diameter of the member of figure 7 can be slightly smaller than that of the member of figure 8. Even this embodiment comprises a multiplicity of signaling members 5 which, being opaque to X-rays, are useful for localizing the endoprosthesis during its positioning and the possible length extension thereof.

Now, with reference to figures from 10 to 13, there is shown that the diameter of the endoprosthesis according to the present invention or of another intrahepatic endoprosthesis of a known type can be reduced, once the endoprosthesis has already been inserted into the patient's liver, by means of cross-section reducers which comprise a tube 6 of biocompatible tissue joined to a tubular net 7 and having both end portions 8, 9 flared and coated by the tissue tube 6. Also these cross-section reducers can comprise a multiplicity of signaling elements 5 which are opaque to X-rays. Particularly, in the cross-section reducer according to the second embodiment of the present invention the end portions 8, 9 are provided with a length such that they can fit together and the reducer itself takes on the shape of an hour-glass. Obviously, the length and minimum diameter of the cross-section reducer can very as a function of the desired artificial stenosis effect.

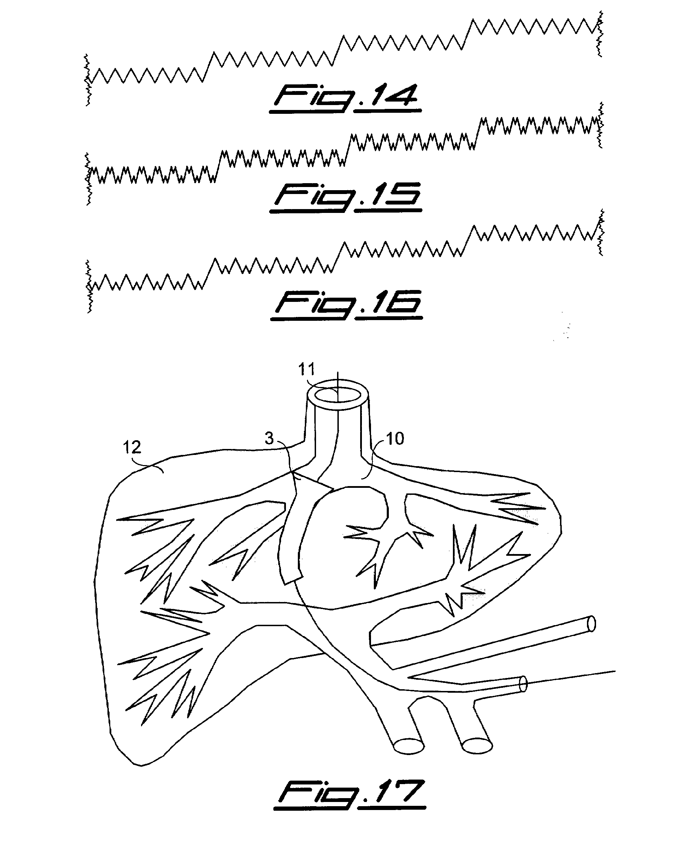

Then, with reference to figures 14 to 16, tubular nets 2 and 7 of the endoprostheses or of the cross-section reducers according to the present invention are preferably obtained by zigzag bending a single metal wire so as to form a substantially triangular wave having alternated crests and valleys of equal heights as in figure 14 or different heights as in figures 15 and 16. Further, the lower and upper profile of such a triangular wave are provided with a whole periodical increase of the height, so that the wire can be easily winded following a helicoidal profile, thus obtaining the tubular nets. For this purpose, the crests and valleys positioned along the external profiles of the triangular wave are connected, for example by means of microscopical sutures, to the valleys and crests of the adjacent portions comprised in a wave period respectively, as well as fixed to tissue tube 1 or 6, still by means of said sutures.

In further embodiments of the present invention the crests can be fixed to the valleys not through microscopic sutures, but by means of weldings with supply of metallic material preferably equal to that of the whole tubular net, for example the same nitinol. By this measure a slightly harder tubular net is obtained, but still flexible and elastical.

In use, before being inserted into the patient's liver, the endoprosthesis according to the present invention is compressed and positioned in a known way inside a suitable tubular container, which is useful for taking the endoprosthesis in the desired position. Once taken in position by means of a pilot wire and with the help of signals 5 for radiological control, the endoprosthesis is removed from the tubular container, thus being free to expand, possibly with the help of a ball catheter.

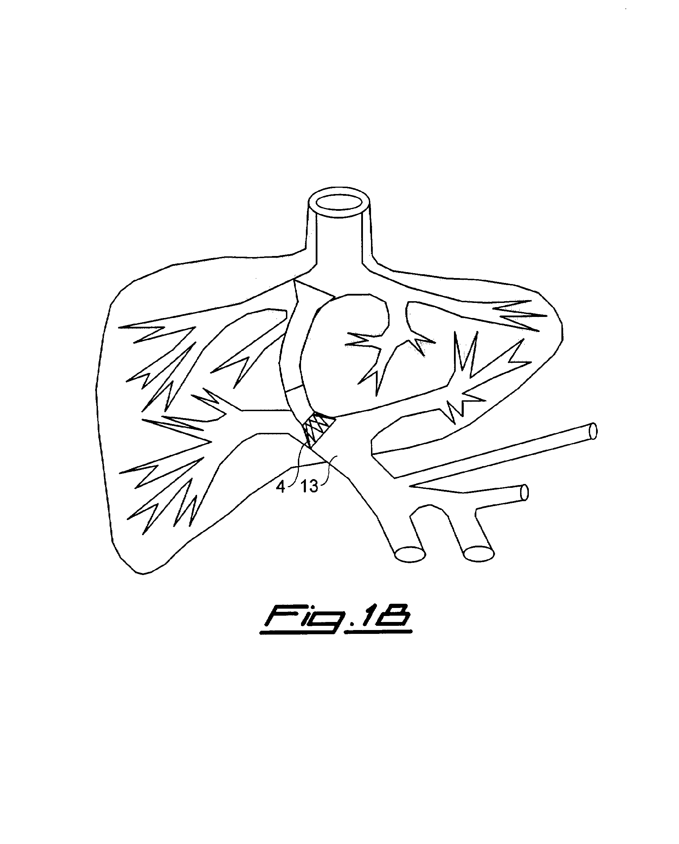

Now, with reference to figures 17 and 18, there is shown that in order to position an endoprosthesis according to the present invention divided in two members as in the fourth embodiment, first the member of figure 7 is compressed in a tubular container, is placed with the flared end 3 at a suprahepatic branch 10 by means of a pilot wire 11 which passes through the liver 12 of the patient, and is then expanded by the above mentioned known procedure. Now, the same operations are carried out for the other member of figure 8, which is passed through tube 1a of the member of figure 7 already expanded. The element of figure 8 is in turn expanded so that the resulting telescopical endoprosthesis at the end occupies the whole hepatic means with the flared end 4 at a portal branch 13, as shown in figure 18.

Further variants and additions can be made by those which are skilled in the art to the above described and illustrated embodiments by remaining within the scope of the invention itself.

## Claims

1. Intrahepatic endoprosthesis comprising at least one tubular net (2), characterized in that the end portions (3, 4) of said tubular net (2) are outwardly flared and that at least one end portion (3) and one portion of said tubular net (2) included between said end portions (3, 4) are joined to a tube (1; 1a, 1b) of biocompatible tissue having substantially the same diameter of said tubular net (2).

2. lntrahepatic endoprosthesis according to the preceding claim, characterized in that the tubular net (2) is arranged inside the tube (1; 1a, 1b) of biocompatible tissue.

3. Intrahepatic endoprosthesis according to claim 1, characterized in that the tubular net (2) is arranged outside the tube (1) of biocompatible tissue.

4. Intrahepatic endoprosthesis according to one of the preceding claims, characterized in that it includes two members which can be telescopically coupled, both comprising a tube (1a, 1b) of biocompatible tissue, which is joined to a tubular net and is provided with a single end portion (3, 4) outwardly flared.

5. Intrahepatic endoprosthesis according to one of the preceding claim, characterized in that the weft of the meshes at the end portions (3, 4) of the tubular net (2) is wider than the weft of the meshes which are included between the portions themselves.

6. Cross-section reducer which can be used in combination with an endoprosthesis according to one of the preceding claims, characterized in that it comprises a tube (6) of biocompatible material joined to a tubular net (7) and having both end portions (8, 9) flared and coated by the tissue tube (6).

7. Cross-section reducer according to the preceding claim, characterized in that the end portions (8, 9) fit together and the reducer is shaped as an hour-glass.

8. Intrahepatic endoprosthesis or cross-section reducer according to one of the preceding claims, characterized in that said tubular net (2, 7) is made in a nickel and titanium alloy.

9. lntrahepatic endoprosthesis or cross-section reducer according to one of the preceding claims, characterized in that said tubular net (2, 7) comprises at least one metal wire, which is bent so as to form a substantially triangular wave, is winded along a helicoidal profile and is provided with crests and valleys connected to each other, wherein the lower profile and upper profile of said triangular wave are provided with an integral periodical height increase.

10. Intrahepatic endoprosthesis or cross-section reducer according to the preceding claim, characterized in that said metal wire is bent so as to form a substantially triangular wave having alternated crests and valleys of two different heights.

11. Intrahepatic endoprosthesis or cross-section reducer according to claim 9 or 10, characterized in that said crests and valleys are welded to each other.

12. Intrahepatic endoprosthesis or cross-section reducer according to one of the preceding claims, characterized in that a multiplicity of signaling members (5) opaque to X-rays is arranged close to the end portions (3, 4, 8, 9).
